# FASCICULE DE BREVET EUROPEEN

(11) **EP 2 462 902 B1**
(45) Date de publication et mention de la délivrance du brevet: **04.09.2013**
(21) Numéro de dépôt: 11354076.9
(22) Date de dépôt: 09.12.2011
(51) Int. Cl.: A61F 2/78, A61F 2/50, A61F 2/00

(54) **Enveloppe d'habillage pour prothèse et procédé de fabrication d'une telle enveloppe**
Verkleidungshülle für Prothese, und Herstellung einer solchen Hülle
Covering envelope for a prosthesis and method for manufacturing such an envelope

(30) Priorité: 10.12.2010 FR 1004813
(43) Date de publication de la demande: 13.06.2012
(73) Titulaire: Chabloz, Pierre, 38450 Saint Georges de Commiers (FR)
(72) Inventeur: Chabloz, Pierre, 38450 Saint Georges de Commiers (FR)
(74) Mandataire: Gris, Sébastien

(56) Documents cités:
- WO-A1-2008/044052
- WO-A1-2010/070296
- DE-A1- 4 125 635
- DE-C- 836 393
- DATABASE WPI Thomson Scientific, London, GB; AN 1978-36136A XP002653813, & SU 560 607 A1 5 juin 1977 (1977-06-05)

## Description

### Domaine technique de l'invention

L'invention concerne une enveloppe d'habillage pour prothèse d'un membre, formée par une paroi ayant une surface externe et une surface interne délimitant une cavité destinée à recevoir la prothèse, ladite enveloppe étant formée par un matériau composite comportant un tissu de renfort noyé dans une matrice polymère.

L'invention concerne également un procédé de fabrication d'une telle enveloppe.

### État de la technique

Les prothèses sont utilisées pour remplacer un ou plusieurs organes d'un individu. Parmi les prothèses, on distingue les exo-prothèses portées sur le corps de l'individu de façon amovible par opposition à une endoprothèse destinée à être implantée dans le corps de l'individu.

L'exo-prothèse remplace l'extrémité d'un membre sectionné ou amputé comme un bras ou une jambe et est adaptée au membre résiduel ou moignon. L'exo-prothèse comporte un ensemble de pièces et de mécanismes fonctionnels permettant l'adaptation de l'exo-prothèse sur le membre résiduel. L'exo-prothèse peut également être articulée pour remplacer un membre manquant articulé comme une prothèse fémorale ou une prothèse de bras.

L'exo-prothèse doit être fonctionnelle mais également esthétique. En effet, l'esthétique d'une exo-prothèse est un problème qui préoccupe les amputés, spécialement pour les membres inférieur et supérieur. Ainsi, les prothèses complètes de membres inférieure et supérieure sont recouvertes par un revêtement souple, généralement en mousse, donnant à la prothèse la forme du membre manquant en s'inspirant le plus possible de l'esthétique du membre inférieur ou supérieur opposé ou d'une photographie réalisée avant amputation pour un amputé bilatéral.

Pour les prothèses complètes articulées, le revêtement doit être capable d'accompagner la flexion de l'articulation sans se déchirer ni freiner les mouvements. Cette contrainte est particulièrement importante pour les prothèses fémorales intervenant dans le processus de déplacement de l'individu appareillé. Le revêtement doit notamment être capable d'accompagner la flexion utile du genou prothétique qui couvre une grande amplitude. Le mouvement de flexion peut couvrir un angle allant de 0° à environ 40° lors de la marche et un angle allant de 0° à d'environ 110° pour une position assise.

Des bas cosmétiques ou bas de recouvrement peuvent être utilisés comme enveloppe d'habillage de la prothèse d'un membre inférieur afin de protéger et améliorer l'aspect esthétique extérieur de la prothèse. Les bas cosmétiques ou bas de recouvrement sont conformés pour épouser la forme de la prothèse et présentent une surface externe et une surface interne. La surface interne est mise en contact avec le revêtement de la prothèse.

Lorsque le membre résiduel est appareillé avec la prothèse du membre inférieur, le bas de recouvrement ou cosmétique est enfilé sur la prothèse pour revêtir entièrement la prothèse. Le revêtement de la prothèse du membre inférieur est ainsi protégé et, également, habillé afin de lui donner un aspect extérieur plus esthétique, par exemple, par le choix de la matière et de la couleur du bas de recouvrement ou cosmétique.

À l'heure actuelle, il existe des bas de recouvrement ou cosmétiques en tissu qui n'entravent, ni ne freinent les mouvements de la prothèse d'un membre inférieur, notamment le mouvement d'un genou prothétique. Néanmoins, ces bas en tissu ne confèrent pas à la prothèse une apparence et un touché similaires à ceux de la peau d'un individu.

Plus récemment, des bas de recouvrement ou cosmétiques en silicone ont été proposés pour reproduire parfaitement l'aspect et la couleur de la peau. Les bas en silicone présentent néanmoins le désavantage de manquer d'élasticité et sont difficiles à enfiler sur la prothèse. Dans le cas de l'habillage d'une prothèse fémorale, le mouvement pendulaire de la prothèse munie d'un tel bas en silicone est fortement gêné et la présence du bas en silicone restreint l'amplitude de la flexion du genou prothétique. Par ailleurs, la flexion et l'extension répétées du genou prothétique entraînent à l'usage une déformation du bas en silicone, localisée au niveau du genou. Cette déformation forme, notamment, des plis disgracieux et inesthétiques.

Le document WO2010/070296 décrit une enveloppe d'habillage pour prothèse selon le préambule de la revendication 1, notamment pour une prothèse de main, formée par une paroi ayant une surface externe et une surface interne délimitant une cavité destinée à recevoir ladite prothèse, la paroi étant formée par un matériau composite comportant un tissu de renfort noyé dans une matrice polymère.

### Objet de l'invention

L'invention a pour but une enveloppe d'habillage pour prothèse remédiant aux inconvénients de l'art antérieur.

Plus particulièrement, l'invention a pour but une enveloppe d'habillage pour prothèse facile à mettre en place sur la prothèse et protégeant le revêtement de la prothèse sans déformer ledit revêtement ni entraver les mouvements prothétiques.

L'invention a également pour but une enveloppe d'habillage pour prothèse ayant une surface externe imitant la texture et l'apparence de la peau d'un individu.

Selon l'invention et comme il est defini dans la revendication 1, ce but est atteint par le fait que :
- la paroi a une épaisseur inférieure à 0,5mm
- le matériau composite a un taux d'allongement élastique supérieur ou égal à 200%,
- le tissu de renfort (10) est formé par des fibres textiles ayant une finesse comprise entre 20 deniers et 30 deniers.

Selon un mode de réalisation préférentiel, les fibres textiles sont en soie, polyamide, élasthanne et/ou coton.

L'invention a également pour but un procédé de fabrication d'une telle enveloppe d'habillage, facile à mettre en oeuvre, peu coûteux et industrialisable. L'objet de l'invention concerne un procédé de fabrication d'une enveloppe d'habillage adaptée aux exo-prothèses, en particulier, aux prothèses articulées permettant d'obtenir de façon reproductible une enveloppe d'habillage ayant un aspect externe, en particulier une texture et une couleur, proche du membre manquant.

Selon l'invention et comme il est défini dans la revendication 6, ce but est atteint par un procédé de fabrication d'une telle enveloppe d'habillage pour prothèse d'un membre qui comporte les étapes successives suivantes :
- réalisation d'un moule positif ayant la forme dudit membre,
- recouvrement du moule positif avec le tissu de renfort conformé pour s'ajuster à la surface dudit moule et comportant des fibres textiles ayant une finesse comprise entre 20 deniers et 30 deniers,
- formation de la paroi de l'enveloppe d'habillage sur le moule positif par application d'au moins une couche d'élastomère sur le tissu de renfort en imprégnant les fibres textiles dudit tissu puis réticulation, la surface externe de ladite paroi étant en contact avec la surface dudit moule et,
- obtention de l'enveloppe d'habillage par démoulage de ladite enveloppe et retournement de la paroi.

Selon un mode de réalisation préférentiel, le démoulage de l'enveloppe d'habillage et le retournement de la paroi sont effectués en une seule étape.

Selon un développement de l'invention, l'élastomère est choisi parmi les élastomères de silicone.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :
- La figure 1 représente, schématiquement et en vue de face, une prothèse fémorale, montée sur un membre résiduel, munie d'un bas de recouvrement selon un mode de réalisation particulier de l'invention,
- La figure 2 représente, schématiquement et en coupe, un bas de recouvrement selon un mode de réalisation particulier de l'invention,
- les figures 3 à 6, représentent, schématiquement et en perspective, différentes étapes d'un procédé de fabrication d'un bas de recouvrement selon la figure 2,
- la figure 7 représente, schématiquement et en coupe, un agrandissement d'une paroi d'un bas de recouvrement selon un autre mode de réalisation particulier de l'invention.

### Description de modes particuliers de réalisation

L'enveloppe d'habillage selon l'invention est destinée à revêtir une prothèse d'un membre lorsque la prothèse est portée par un individu à la place du membre manquant. L'enveloppe d'habillage est, avantageusement, destinée à revêtir une prothèse d'un membre inférieur ou supérieur.

L'enveloppe d'habillage est, particulièrement, adaptée pour habiller une prothèse articulée comme une prothèse fémorale ou une prothèse de bras.

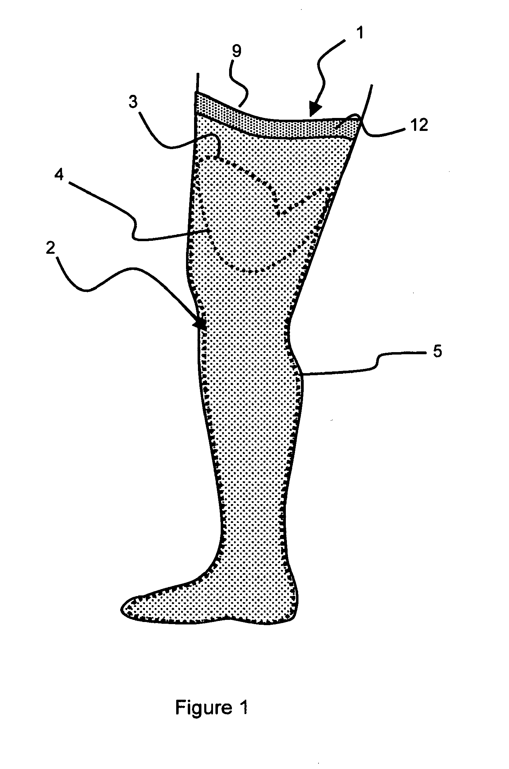

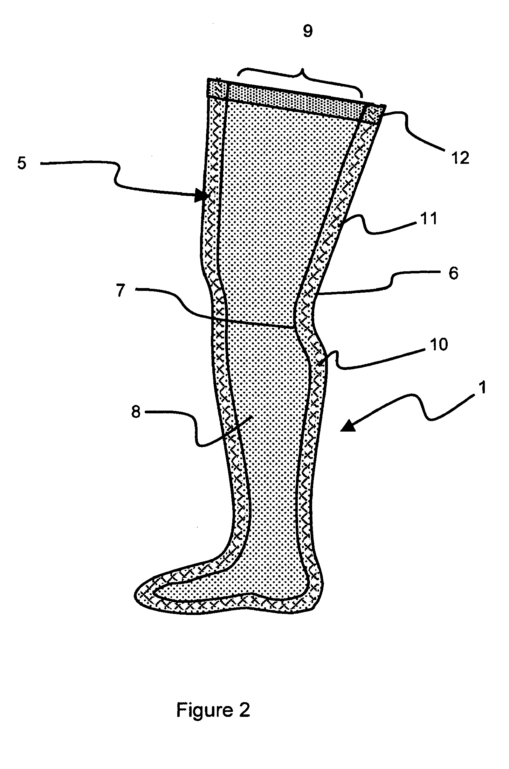

Selon un mode particulier de réalisation représenté aux figures 1 et 2, une enveloppe d'habillage constitue un bas de recouvrement 1 pour une prothèse d'un membre inférieur. Comme représenté à la figure 1, la prothèse du membre inférieur est, par exemple, une prothèse fémorale 2 articulée. La prothèse fémorale 2 comporte un revêtement externe 3 ayant la forme du membre inférieur manquant c'est-à-dire, dans ce cas, d'une jambe. La prothèse fémorale 2 est montée sur un membre résiduel 4 et est munie du bas de recouvrement 1. Le bas de recouvrement 1 est conformé pour épouser la forme de la prothèse fémorale 2.

Comme représenté à la figure 2, le bas de recouvrement 1 est formé par une paroi 5 ayant une surface externe 6 et une surface interne 7. La surface interne 7 délimite une cavité 8 destinée à recevoir la prothèse fémorale 2 et, éventuellement, une partie du membre résiduel 4.

Le bas de recouvrement 1 présente une ouverture 9 aménagée dans la paroi 5, pour permettre l'introduction de la prothèse fémorale 2. La paroi 5 est conformée pour épouser la forme de la prothèse fémorale 2 de manière à ce que la surface interne 7 est en contact avec la prothèse fémorale 2 et, optionnellement, la peau du membre résiduel 4.

La paroi 5 est formée par un matériau composite comportant un tissu de renfort 10 noyé dans une matrice polymère 11. En particulier, la paroi 5 du bas de recouvrement 1 est uniquement constituée par le matériau composite.

La paroi 5 du bas de recouvrement 1 a une épaisseur inférieure à 5mm, avantageusement, inférieure ou égale à 4mm, de préférence, inférieure ou égale à 3mm. L'épaisseur est, de préférence, uniforme sur les zones visibles.

Par ailleurs, la paroi 5 peut être plus épaisse sur certaines zones pour former des renforts comme, par exemple, sur la zone recouvrant le talon ou les orteils du pieds de la prothèse fémorale 2.

La matrice polymère 11 est, de préférence, une résine silicone.

Les fibres textiles assurent le renfort du bas de recouvrement 1 et doivent être suffisamment fines et souples pour limiter l'épaisseur du bas de recouvrement 1 et éviter la formation de plis disgracieux lors des mouvements prothétiques. Les fibres textiles sont, de préférence, en soie, polyamide, élasthanne et/ou coton. Le tissu de renfort 10 est formé par des fibres textiles ayant une finesse comprise entre 20 deniers et 30 deniers. Les valeurs de 20 deniers et 30 deniers correspondent, respectivement, aux valeurs de 2,20 tex et 3,35 tex. Le tissu de renfort 10 a, avantageusement, un taux d'allongement élastique supérieur ou égal à 200%, de préférence, compris entre 200% et 700%.

Le matériau composite a, avantageusement, une dureté Shore A comprise entre 5 et 15, de préférence, entre 10 et 12. Par ailleurs, le matériau composite a un taux d'allongement élastique supérieur ou égal à 200%, avantageusement, compris entre 200% et 700%, de préférence, entre 200% et 400%.

Comme représenté à la figure 2, le bas de recouvrement 1 peut, avantageusement, comporter une bande de maintien 12 en bordure de l'ouverture 9 de manière à maintenir le bas de recouvrement 1 en place lors des mouvements prothétiques, par exemple, lors de la marche.

Le bas de recouvrement 1 a, avantageusement, une forme imitant la forme présumée de la jambe manquante de l'individu et une longueur permettant de couvrir avantageusement le genou jusqu'à la cuisse. La forme supposée de la jambe manquante est définie en se basant sur la jambe opposée ou à partir d'un modèle de jambe standard dans le cas d'une amputation bilatérale. Les dimensions du bas de recouvrement 1 correspondent, de préférence, aux dimensions présumées du membre manquant avec un écart pouvant aller de 0% à 25% par rapport aux dimensions présumées du membre manquant. À titre d'exemple, un bas de recouvrement 1 ayant une longueur de 1 mètre peut être utilisé pour habiller une gamme de prothèses de longueur comprise entre 0,75 mètre et 1,25 mètres. Ainsi, seules quatre tailles de bas de recouvrement 1 suffisent pour couvrir une large gamme de prothèses fémorales 2.

La surface externe 6 de la paroi 5 du bas de recouvrement 1 présente, avantageusement, des motifs en creux et en saillie reproduisant le grain de peau d'un individu (non représentés).

Le bas de recouvrement 1 est utilisé sur le membre résiduel 4 de l'individu équipé par la prothèse fémorale 2. L'utilisation du bas de recouvrement 1 consiste à enfiler le bas de recouvrement 1 sur la prothèse fémorale 2 par l'ouverture 9, comme une chaussette ou un bas de chausse. Le bas de recouvrement 1 est remonté le long de la prothèse fémorale 2 jusqu'à ce qu'elle recouvre la totalité de la prothèse 2. Le bas de recouvrement 1 habille ainsi la prothèse fémorale 2 et, éventuellement, une partie du membre résiduel 4 appareillé. Le bas de recouvrement 1 a des propriétés élastiques et une texture adaptées pour pouvoir glisser sur la prothèse fémorale 2 et s'allonger suffisamment par étirement pour couvrir au moins la prothèse fémorale 2. Les propriétés mécaniques du matériau composite assurent également le maintien du bas de recouvrement 1 en place même lors du processus de déplacement de l'individu appareillé. Le bas de recouvrement 1 peut accompagner la flexion utile du genou prothétique sur une grande amplitude d'angles allant de 0° à 110° grâce, notamment, aux propriétés mécanique et élastique du matériau composite et à la sélection de la gamme d'épaisseur de la paroi 5.

Comme représenté à la figure 1, le bas de recouvrement 1 peut avantageusement être allongé au-delà de la prothèse fémorale 2, pour envelopper également une partie du membre résiduel 4 de l'individu.

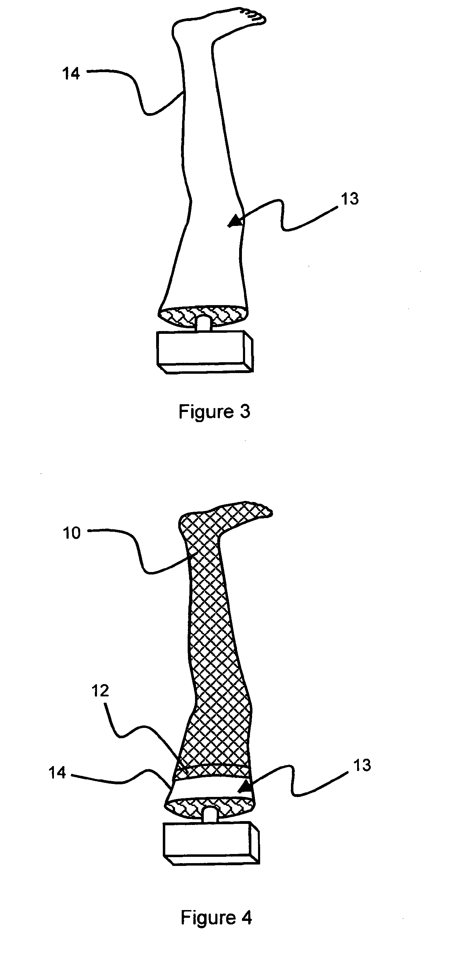

Selon un mode de réalisation particulier représenté aux figures 3 à 6, le bas de recouvrement 1 de la prothèse fémorale 2 décrit ci-dessus est fabriqué selon une technique de moulage par contact ou projection à partir d'un moule positif 13.

Comme représenté à la figure 3, un procédé de fabrication du bas de recouvrement 1 comporte une étape de réalisation du moule positif 13 ayant la forme du membre manquant c'est-à-dire la forme d'une jambe pour une prothèse fémorale 2. La réalisation du moule positif 13 est effectuée selon tout procédé connu et de manière à se rapprocher le plus possible de la forme de la jambe manquante. À titre d'exemple, le moule positif 13 peut être réalisé en résine ou, de préférence, en plâtre ou en métal, à partir d'un moule négatif de jambe standard puis travaillé pour apporter les détails lui donnant un aspect personnalisé.

La surface 14 du moule positif 13 définit l'aspect externe du bas de recouvrement 1. La surface 14 du moule positif 13 peut, avantageusement, présenter des motifs en saillie et en creux, reproduisant en négatif le grain de la peau d'un individu. Ainsi, les motifs en saillie et en creux sont réalisés de manière à pouvoir être transférés à la surface externe 6 de la paroi 5 du bas de recouvrement 1, par une technique de moulage par contact ou projection du moule positif 13.

Comme représenté à la figure 4, après réalisation du moule positif 13, on recouvre le moule positif 13 avec un tissu de renfort 10 conformé pour s'ajuster à la surface 14 du moule positif 13.

Le tissu de renfort 10 comporte des fibres textiles ayant une finesse comprise entre 20 deniers et 30 deniers. Le tissu de renfort 10 peut, avantageusement, être un bas de chausse formé de fibres textiles en soie, polyamide, élasthanne ou coton. Le bas de chausse peut également être constitué par un mélange de différentes fibres textiles en soie, polyamide, élasthanne et/ou coton. On entend par bas de chausse, une chaussette fine et longue qui gaine la jambe depuis le pied jusqu'en haut de la cuisse. Le bas de chausse peut présenter une bande de maintien 12 constituant un moyen supplémentaire de maintien du bas de recouvrement 1 sur l'individu équipé de la prothèse fémorale 2.

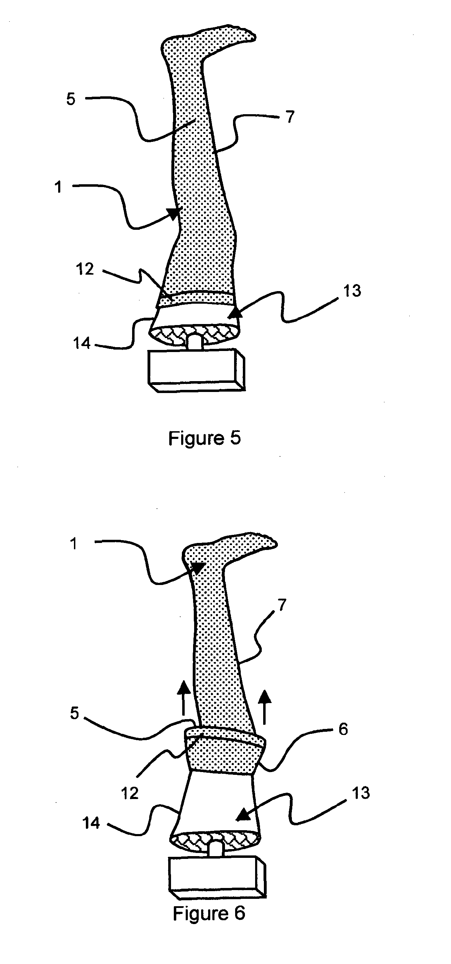

Comme représenté à la figure 5, la paroi 5 du bas de recouvrement 1 est ensuite formée sur le moule positif 13 de manière à ce que la surface externe 6 de la paroi 5 est en contact avec au moins une partie de la surface 14 du moule positif 13. La partie recouverte par la surface externe 6 de la paroi 5 doit au moins correspondre à la surface du revêtement externe 3 de la prothèse fémorale 2. La formation de la paroi 5 est réalisée par application d'au moins une couche d'élastomère sur le tissu de renfort 10 en imprégnant les fibres textiles dudit tissu 10 puis réticulation.

L'application de la couche d'élastomère peut être effectuée par pose manuelle ou projection selon tout procédé connu.

La couche d'élastomère appliquée doit avoir une épaisseur suffisante pour imprégner les fibres textiles du tissu de renfort 10 et recouvrir la totalité du tissu de renfort 10. La couche d'élastomère a une épaisseur inférieure à 5mm, avantageusement inférieure ou égale à 4mm, de préférence inférieure ou égale à 3mm. La couche d'élastomère adopte la forme du moule positif 13, en particulier, les motifs en creux et en saillie destinés à reproduire l'aspect de la peau de la jambe manquante.

La couche d'élastomère peut être transparente ou colorée.

La réticulation est réalisée selon tout procédé connu, par exemple, par réticulation chimique, thermique ou radicalaire par irradiation UV. La réticulation provoque la polymérisation de l'élastomère pour former la matrice polymère 11 du matériau composite. Les fibres textiles du tissu de renfort 10, imprégnées par l'élastomère, sont ainsi emprisonnées et noyées dans la matrice polymère 11. À titre d'exemple, la réticulation est une vulcanisation à froid pour un élastomère silicone. La prise de l'élastomère silicone se produit par simple contact avec l'humidité de l'air.

L'élastomère est, avantageusement, un élastomère thermoplastique de type silicone (SI), polyuréthane (PU) ou éthylène-acétate de vinyle (EVA) ayant une dureté Shore A comprise entre 5 et 15, de préférence, entre 10 et 12 et un taux d'allongement élastique supérieur ou égal à 200%, avantageusement, compris entre 200% et 700%, de préférence, entre 200% et 400%. L'élastomère est choisi en fonction de la nature des fibres textiles du tissu de renfort 10, pour éviter toute ségrégation entre les fibres textiles et la matrice polymère 11 formée après réticulation. Le phénomène de ségrégation entraîne le décollement de la matrice polymère 11 des fibres textiles et la formation de plusieurs phases dans le matériau composite.

L'élastomère est, de préférence, choisi parmi les élastomères de silicone. Les élastomères de silicone permettent une bonne imprégnation des fibres textiles en soie, polyamide, élasthanne et/ou coton, sans ségrégation.

Après réticulation, la couche d'élastomère forme la matrice polymère 11 du matériau composite dans laquelle le tissu de renfort 10 est noyé.

Comme représenté à la figure 6, le bas de recouvrement 1 ainsi formé est disposé à l'envers sur le moule positif 13. La surface externe 6 de la paroi 5 du bas de recouvrement 1 est en contact avec la surface 14 du moule positif 13.

Le bas de recouvrement 1 est, ensuite, obtenu par démoulage du bas de recouvrement 1 et retournement de la paroi 5. Selon un mode de réalisation préférentiel, le démoulage du bas de recouvrement 1 et le retournement de la paroi 5 du bas de recouvrement 1 sont effectués en une seule étape. Le démoulage est réalisé en retournant la paroi 5 (flèches verticales à la figure 6). Le retournement a une action, double et simultanée, d'extraction dudit bas 1 du moule positif 13 et de retournement de la paroi 5 permettant ainsi de libérer la surface externe 6 et d'obtenir le bas de recouvrement 1 sous sa forme définitive d'utilisation c'est-à-dire avec une surface interne délimitant la cavité 8 et une surface externe visible.

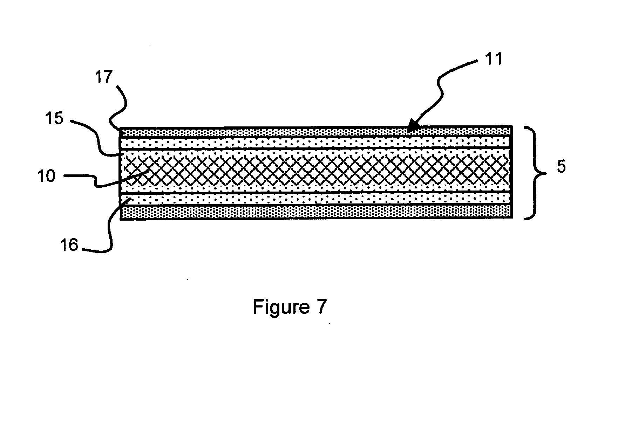

Selon un mode de réalisation préférentiel, le procédé de fabrication est identique au mode de réalisation décrit ci-dessus à l'exception du fait que la formation de la paroi 5 du bas de recouvrement 1 est effectuée par applications successives de plusieurs couches d'un même élastomère ou d'élastomères différents sur le tissu de renfort 10. Comme représenté à la figure 7, le bas de recouvrement 1 a une paroi 5 formée par un stratifié de première, seconde et troisième couches d'élastomère(s), respectivement, 15, 16 et 17 qui recouvrent le tissu de renfort 10.

Une réticulation au moins partielle est réalisée après chaque application. Dans le cas d'une vulcanisation à froid, un temps d'attente est nécessaire entre chaque application, pour permettre la réticulation au moins partielle de la couche d'élastomère appliquée. À titre d'exemple, après l'application d'une première couche 15 d'élastomère silicone bi-composants FA 109-12 commercialisé par la société Prevent Silicones, ayant une dureté Shore A de 10/12 et un taux d'allongement de 700%, on attend entre 30min et 2h pour appliquer la seconde couche 16 du même élastomère silicone FA 109-12 et entre 30min et 2h pour appliquer la troisième couche d'un élastomère silicone coloré 17. La réticulation est, avantageusement, partielle de manière à optimiser l'adhésion des différentes couches 15, 16 et 17 entre-elles. Ainsi, la première couche d'élastomère 15 reste collante pour adhérer d'avantage à la seconde couche d'élastomère 16 lors de l'application de cette dernière et la seconde couche d'élastomère 16 reste collante pour adhérer d'avantage à la troisième couche d'élastomère 17 lors de l'application de la troisième couche d'élastomère 17. Après réticulations de chaque couches 15, 16 et 17, les première, seconde et troisième couches d'élastomère(s), respectivement 15, 16 et 17, forment la matrice polymère 11 du matériau composite constituant la paroi 5. La matrice polymère 11 enrobe les fibres textiles du tissu de renfort 10 du matériau composite.

Les première, seconde et troisième couches d'élastomère(s), respectivement 15, 16 et 17, doivent constituées une épaisseur suffisante pour imprégner les fibres textiles du tissu de renfort 10 et recouvrir la totalité du tissu de renfort 10. Chaque première, seconde ou troisième couche d'élastomère, respectivement 15, 16 et 17 a une épaisseur, de préférence, inférieure ou égale à 0,2mm.

On obtient un matériau composite constitué par un tissu de renfort 10 noyé dans une matrice polymère 11. Le matériau composite est, de préférence, extensible de façon élastique dans au moins une direction. Le matériau composite est, avantageusement, extensible dans le sens de la longueur jusqu'à au moins 200% en plus de sa longueur initiale, avantageusement, entre 200% et 700% de sa longueur initiale, de préférence, entre 200% et 400% de sa longueur initiale. Le matériau composite est, de préférence, extensible de façon élastique dans le sens de la largeur du bas de recouvrement 1 jusqu'à au moins 200% en plus de sa longueur initiale, avantageusement, entre 200% et 700% de sa longueur initiale, de préférence, entre 200% et 400% de sa longueur initiale.

De façon surprenante, le matériau composite ainsi réalisé possède des propriétés mécaniques et une texture améliorées que le tissu de renfort 10 et la matrice polymère 11 seuls ne possèdent pas. Le matériau composite confère au bas de recouvrement 1 des propriétés d'étanchéité, d'élasticité et une texture améliorée. Le bas de recouvrement 1 a, notamment, une surface interne 7, lisse et glissante, qui facilite la mise et l'enlèvement du bas de recouvrement 1 sur la prothèse fémorale 2. Par ailleurs, le bas de recouvrement 1 a un pouvoir gainant exerçant une pression suffisante pour le maintenir en place sur la prothèse fémorale 2, sans toutefois déformer le revêtement externe 3. Par ailleurs, le bas de recouvrement 1 n'entrave pas les mouvements prothétiques, notamment, les mouvements pendulaires de la prothèse fémorale 2.

L'enveloppe d'habillage selon l'invention est remarquable en ce qu'elle présente des propriétés mécaniques améliorées, adaptées à l'habillage d'une prothèse, en particulier, articulée. Le procédé de fabrication selon l'invention permet d'obtenir une enveloppe d'habillage ayant des caractéristiques particulièrement avantageuses, notamment un pouvoir gainant suffisant pour rester en place sur une prothèse lors des mouvements prothétiques sans toutefois déformer le revêtement de la prothèse, entraver les mouvements prothétiques ni former des plis inesthétiques lors de la flexion du genoux ou du coude.

## Revendications

1. Enveloppe d'habillage pour prothèse (2) d'un membre, formée par une paroi (5) ayant une surface externe (6) et une surface interne (7) délimitant une cavité (8) destinée à recevoir ladite prothèse (2), ladite enveloppe étant formée par un matériau composite comportant un tissu de renfort (10) noyé dans une matrice polymère (11),
**caractérisée en ce que**
- la paroi (5) a une épaisseur inférieure à 0,5mm,
- le matériau composite a un taux d'allongement élastique supérieur ou égal à 200%.
- le tissu de renfort (10) est formé par des fibres textiles ayant une finesse comprise entre 20 deniers et 30 deniers.

2. Enveloppe d'habillage selon la revendication 1, **caractérisée en ce que** les fibres textiles sont en soie, polyamide, élasthanne et/ou coton.

3. Enveloppe d'habillage selon l'une des revendications 1 et 2, **caractérisée en ce que** la matrice polymère (11) est une résine silicone.

4. Enveloppe d'habillage selon l'une quelconque des revendications 1 à 3, **caractérisée en ce que** la surface externe (6) de la paroi (5) présente des motifs en creux et en saillie reproduisant le grain de peau d'un individu.

5. Enveloppe d'habillage selon l'une quelconque des revendications 1 à 4, **caractérisée en ce qu'**elle constitue un bas de recouvrement (1).

6. Procédé de fabrication d'une enveloppe d'habillage pour prothèse (2) d'un membre selon l'une quelconque des revendications 1 à 5, **caractérisé en ce qu'**il comporte les étapes successives suivantes :
- réalisation d'un moule positif (13) ayant la forme dudit membre,
- recouvrement du moule positif (13) avec le tissu de renfort (10) conformé pour s'ajuster à la surface (14) dudit moule (13) et comportant des fibres textiles ayant une finesse comprise entre 20 deniers et 30 deniers,
- formation de la paroi (5) de l'enveloppe d'habillage (1) sur le moule positif (13) par application d'au moins une couche d'élastomère sur le tissu de renfort (10) en imprégnant les fibres textiles dudit tissu (10) puis réticulation, la surface externe (6) de ladite paroi (5) étant en contact avec la surface (14) dudit moule (13) et,
- obtention de l'enveloppe d'habillage (1) par démoulage de ladite enveloppe (1) et retournement de la paroi (5).

7. Procédé selon la revendication 6, **caractérisé en ce que** l'élastomère est choisi parmi les élastomères de silicone.

8. Procédé selon les revendications 6 et 7, **caractérisé en ce que** le démoulage de l'enveloppe d'habillage (1) et le retournement de la paroi (5) sont effectués en une seule étape.

9. Procédé selon l'une quelconque des revendications 6 à 8, **caractérisé en ce que** la surface (14) du moule positif (13) présente des motifs en saillie et en creux reproduisant en négatif le grain de la peau d'un individu.

10. Procédé selon l'une quelconque des revendications 6 à 9, **caractérisé en ce que** la formation de la paroi (5) de l'enveloppe d'habillage (1) est effectuée par applications successives de plusieurs couches d'un même élastomère ou d'élastomères différents (15, 16 et 17) sur le tissu de renfort (10) et **en ce qu'**une réticulation au moins partielle est réalisée après chaque application.

## Patentansprüche

1. Anziehhülle für eine Gliedmaßenprothese (2), die von einer Wand (5) mit einer Außenseite (6) und einer Innenseite (7) gebildet wird, die einen Hohlraum (8) begrenzen, der die Prothese (2) aufnehmen soll, wobei die Hülle aus einem Verbundwerkstoff besteht, der einen Verstärkungsstoff (10) umfasst, der in eine Polymermatrix (11) eingebettet ist, **dadurch gekennzeichnet, dass**
- die Wand (5) eine Dicke von unter 0,5 mm hat,
- das Verbundmaterial einen elastischen Längungsgrad von über oder gleich 200 % hat,
der Verstärkungsstoff (10) von Textilfasern mit einer Feinheit von 20 bis 30 Denier gebildet wird.

2. Anziehhülle nach Anspruch 1, **dadurch gekennzeichnet, dass** die Textilfasern aus Seide, Polyamid, Elasthan und/oder Baumwolle sind.

3. Anziehhülle nach einem der Ansprüche 1 und 2, **dadurch gekennzeichnet, dass** die Polymermatrix (11) ein Silikonharz ist.

4. Anziehhülle nach einem der Ansprüche 1 bis 3, **dadurch gekennzeichnet, dass** die Außenseite (6) der Wand (5) erhabene und vertiefte Motive aufweist, die das Hautbild eines Menschen nachbilden.

5. Anziehhülle nach einem der Ansprüche 1 bis 4, **dadurch gekennzeichnet, dass** sie einen Bekleidungsstrumpf (1) bildet.

6. Herstellungsverfahren für eine Anziehhülle für eine Gliedmaßenprothese (2) nach einem der Ansprüche 1 bis 5, **dadurch gekennzeichnet, dass** es die folgenden aufeinanderfolgenden Schritte umfasst:
- Herstellung einer Positivform (13), die die Form der genannten Gliedmaße hat,
- Bedeckung der Positivform (13) mit dem Verstärkungsstoff (10), der so geformt ist, dass er sich der Oberfläche (14) der Form (13) anpasst und Textilfasern mit einer Feinheit von 20 bis 30 Denier umfasst,
- Ausbildung der Wand (5) derAnziehhülle (1) auf der Positivform (13) durch Auftragen mindestens einer Elastomerschicht auf den Verstärkungsstoff (10) und damit Imprägnieren der Textilfasern mit diesem Verstärkungsstoff (10) unter anschließender Vernetzung, wobei die Außenseite (6) der Wand (5) in Kontakt mit der Oberfläche (14) der Form (13) ist, und
- Erhalt der Anziehhülle (1) durch Lösen derselben (1) aus der Form und Umstülpen der Wand (5).

7. Verfahren nach Anspruch 6, **dadurch gekennzeichnet, dass** das Elastomer aus den Silikonelastomeren ausgewählt ist.

8. Verfahren nach Anspruch 6 und 7, **dadurch gekennzeichnet, dass** das Lösen der Anziehhülle (1) aus der Form und das Umstülpen der Wand (5) in einem Schritt erfolgen.

9. Verfahren nach einem der Ansprüche 6 bis 8, **dadurch gekennzeichnet, dass** die Oberfläche (14) der Positivform (13) erhabene und vertiefte Motive aufweist, die das Hautbild eines Menschen in Negativform nachbilden.

10. Verfahren nach einem der Ansprüche 6 bis 9, **dadurch gekennzeichnet, dass** die Herstellung der Wand (5) der Anziehhülle (1) durch aufeinanderfolgendes Auftragen mehrerer Schichten ein und desselben Elastomers oder unterschiedlicher Elastomere (15, 16 und 17) auf den Verstärkungsstoff (10) erfolgt und dadurch, dass nach jedem Aufbringvorgang zumindest eine parzielle Vernetzung durchgeführt wird.

## Claims

1. Covering envelope for a limb prosthesis (2) formed by a wall (5) with an external face (6) and an internal face (7) confining a cavity (8) designed for receiving said prosthesis (2), said envelope being formed by a composite comprising a reinforcing tissue (10) embedded in a polymer matrix (11), **characterized in that**
- the wall (5) has a thickness of less than 0,5 mm,
- the composite material has a degree of elastic elongation of more than or equalling 200 %,
- the reinforcing tissue (10) is made from textile fibres with a fineness of 20 to 30 denier.

2. Covering envelope according to claim 1, **characterized in that** the textile fibres consist of silk, polyamide, elastane and/or cotton.

3. Covering envelope according to one of claims 1 and 2, **characterized in that** the polymer matrix (11) is a silicone resin.

4. Covering envelope according to one of claims 1 to 3, **characterized in that** the external face (6) of the wall (5) has sunken and embossed patterns reproducing the skin structure of an individual.

5. Covering envelope according to one of claims 1 to 4, **characterized in that** it is a covering stocking (1).

6. Method for manufacturing of a covering envelope for a limb prosthesis (2) according to one of claims 1 to 5, **characterized in that** it comprises the following consecutive steps:
- preparation of a positive mould (13) in the form of said limb,
- covering of the positive mould (13) with the reinforcing tissue (10) which is of such shape that it adapts itself to the surface (14) of the mould (13) and comprising textile fibres with a fineness of 20 to 30 denier,
- constitution of the wall (5) of the covering envelope (1) on the positive mould (13) by application of at least one elastomer layer on the reinforcing tissue (10) by soaking the textile fibres of said tissue (10) and subsequent reticulation, the external face (6) of said wall (5) being in contact with the surface (14) of said mould (13), and
- achievement of the covering envelope (1) by removal of said envelope (1) from the mould and turning out the wall's inside (5).

7. Procedure according to claim 6, **characterized in that** the elastomer is selected from silicone elastomers.

8. Procedure according to claims 6 and 7, **characterized in that** removal of the covering envelope (1) from the mould and turning out of the wall's (5) inside are realized in one step.

9. Procedure according to one of claims 6 to 8, **characterized in that** the surface of the positive mould (13) has patterns moulded into or onto it to negatively reproduce the skin structure of an individual.

10. Procedure according to one of claims 6 to 9, **characterized in that** the wall (5) of the covering envelope (1) is obtained by consecutive application of several layers of the same elastomer or of different elastomers (15, 16 and 17) on the reinforcing tissue (10) and **in that** at least a partial reticulation is performed after every application.
